# EUROPÄISCHE PATENTSCHRIFT

(11) **EP 2 816 049 B1**
(45) Veröffentlichungstag und Bekanntmachung des Hinweises auf die Patenterteilung: **30.05.2018**
(21) Anmeldenummer: 13003143.8
(22) Anmeldetag: 20.06.2013
(51) Int. Cl.: C07F 9/38, A61K 6/02, C09J 133/00

(54) **ß-Ketophosphonsäuren und Dentalmateriallen auf deren Basis**
ß-ketophosphonic acids and all dental materials based thereon
Acides ß-cétophosphoniques et matériaux dentaires à base de ceux-ci

(43) Veröffentlichungstag der Anmeldung: 24.12.2014
(73) Patentinhaber: Ivoclar Vivadent AG, 9494 Schaan (LI)
(72) Erfinder: Moszner, Norbert, LI-9493 Mauren (LI); Catel, Yohann, CH-7320 Sargans (CH); Bock, Thorsten, AT-6800 Feldkirch (AT)
(74) Vertreter: Uexküll & Stolberg

(56) Entgegenhaltungen:
- EP-A1- 1 057 468
- DE-A1- 19 918 974
- US-A- 6 160 015
- US-A1- 2005 048 398
- NO, JOO HWAN ET AL: "Lipophilic analogs of zoledronate and risedronate inhibit Plasmodium geranylgeranyl diphosphate synthase (GGPPS) and exhibit potent antimalarial activity", PROCEEDINGS OF THE NATIONAL ACADEMY OF SCIENCES OF THE UNITED STATES OF AMERICA, Bd. 109, Nr. 11, 2012, Seiten 4058-4063, XP002713050, ISSN: 0027-8424
- NORMAN, DEREK D. ET AL: "Autotaxin inhibition: Development and application of computational tools to identify site-selective lead compounds", BIOORGANIC & MEDICINAL CHEMISTRY, Bd. 21, Nr. 17, 2013, Seiten 5548-5560, XP002713051, ISSN: 0968-0896 -& NORMAN, DEREK D. ET AL: "Supporting Information:Autotaxin inhibition: Development and application of computational tools to identify site-selective lead compounds", BIOORGANIC & MEDICINAL CHEMISTRY, Seiten S1-S21, XP002713891,

## Beschreibung

Die Erfindung betrifft Dentalwerkstoffe, die radikalisch polymerisierbare β-Keto-phosphonsäuren enthalten. Diese Dentalwerkstoffe eigenen sich besonders als Adhäsive, Zemente oder Beschichtungsmaterialien.

Radikalisch polymerisierbare Monomere mit aciden Gruppen werden regelmäßig zur Herstellung von Dentalwerkstoffen eingesetzt. Sie verleihen Dentalmaterialien einerseits selbstätzende Eigenschaften, so dass auf eine Säurebehandlung der Zahnoberfläche vor dem Auftrag der Materialien zum Anätzen der Zahnoberfläche und zum Entfernen der sogenannten Schmierschicht verzichtet werden kann. Außerdem verbessern sie durch ionische oder kovalente Wechselwirkungen mit der Zahnsubstanz die Haftung am Zahn.

Das Ätzvermögen der Monomere wird weitgehend durch ihre Acidität bestimmt. Diese nimmt von Sulfonsäuren über acide Phosphate und Phosphonsäuren zu Carbonsäuren hin ab. Derzeit werden hauptsächlich acide Phosphate zur Herstellung von selbstätzenden dentalen Adhäsiven verwendet. Obwohl Sulfonsäuren eine höhere Acidität haben, kommt praktisch nur der 2-Acrylamido-2-methylpropansulfonsäure eine größere Bedeutung zu, da die sonstigen Eigenschaften von Sulfonsäuren, wie z.B. ihrer Polymerisationsfähigkeit und ihrer Bindungsfähigkeit an die natürliche Zahnsubstanz, nicht optimal sind.

Die EP 1 057 468 A1 offenbart dentale Adhäsive, die als acide Komponente phosphatgruppenhaltige Monomere wie Methacryloyloxydecyldihydrogenphosphat (MDP), Methacryloyloxyethylphenylhydrogenphosphat (MEPP) oder Methacryloyloxyethyldihydrogenphosphat (MEP) enthalten. Diese Monomere werden nach wie vor in großem Umfang zur Herstellung von selbstätzenden Dentalwerkstoffen eingesetzt.

Nachteilig an diesen Verbindungen ist, dass sie in wässrigen Lösungen nicht stabil sind. Sowohl ihre Phosphorsäureesterbindungen als auch ihre Methacrylatesterbindungen werden in Anwesenheit von Wasser hydrolytisch gespalten. Die Hydrolyse wird durch die von den aciden Monomeren freigesetzten Protonen beschleunigt. Wasser wird in selbstätzenden Dentalwerkstoffen regelmäßig als Lösungsmittel eingesetzt, da es für die beim Anätzen der Zahnsubstanz stattfindenden ionischen Prozesse erforderlich ist.

Zur Verbesserung der Hydrolysebeständigkeit von selbstätzenden Dentalmaterialien wurden acide Monomere vorgeschlagen, die hydrolytisch stabilere Bindungen zwischen der polymerisierbaren Gruppe und der aciden Gruppe aufweisen.

Die DE 100 18 968 C1 offenbart polymerisierbare Acrylphosphonsäuren mit hoher Hydrolysestabilität. In Phosphonsäuren ist die Phosphorsäureesterbindung durch eine weniger hydrolyseanfällige direkte Bindung zwischen Kohlenstoff und Phosphor ersetzt. Die Acrylphosphonsäuren zeichnen sich weiter dadurch aus, dass die polymerisierbare Gruppe vorzugsweise über Etherstatt Esterfunktionen angebunden ist.

Die DE 102 34 326 B3 betrifft Acrylesterphosphonsäuren wie zum Beispiel 2,4,6-Trimethylphenyl-2-[4-(dihydroxyphosphoryl)-2-oxabutyl]acrylat, das eine besonders hohe Hydrolysestabilität aufweist. Die verbesserte Hydrolysestabilität ist auf sterische Wechselwirkungen zurückzuführen.

Die DE 199 18 974 A1 offenbart (Meth)acrylate von Hydroxyalkylphosphonaten wie 2-Methacryloxyethanphosphonsäure (MAPA), die sich durch eine starke und dauerhafte Anbindung zum Zahnuntergrund auszeichnen sollen. Die Hydrolysestabilität diese Monomere ist allerdings nicht zufriedenstellend.

Die EP 1 169 996 A1 betrifft Dentalmaterialien, die gute Hafteigenschaften und eine hohe Hydrolysestabilität aufweisen sollen. Die Materialien enthalten polymerisierbare Phosphonsäuren wie beispielsweise 1-(2,5-Dimethyl-1,5-hexadienyl)-phosphonsäure (DMHD) und 4-Methacrylamido-4-methyl-pentyl-phosphonsäure (MAMPA). Zumindest DMHD ist für die radikalische Polymerisation wenig geeignet.

Ein Nachteil von Phosphonsäuren gegenüber den aciden Phosphaten ist ihre geringere Acidität und das damit einhergehende geringere Ätzvermögen gegenüber Zahnschmelz und Dentin.

Der Erfindung liegt die Aufgabe zugrunde, Dentalmaterialien mit aciden polymerisierbaren Monomeren zur Verfügung zu stellen, die über eine hohe Hydrolysestabilität in Kombination mit einer hohen Acidität verfügen. Darüber hinaus sollen die Monomere über ein Eigenschaftsprofil verfügen, das für dentale Anwendungen erforderlich ist. Insbesondere sollen sie in polaren Lösungsmitteln und in Mischungen aus polaren Lösungsmitteln und Wasser gut löslich sein, eine hohe Polymerisationsrate bei der radikalischen Polymerisation aufweisen und eine gute Haftung an der Zahnhartsubstanz und insbesondere an Zahnschmelz zeigen.

Die Aufgabe wird erfindungsgemäß durch Dentalmaterialien gelöst, die mindestens eine β-Ketophosphonsäure gemäß der allgemeinen Formel I und mindestens einen Initiator für die radikalische Polymerisation enthalten: 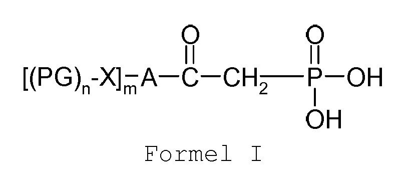 in der
- A =: ein aliphatischer C₁-C₁₈-Rest ist, der durch -O-, -S-, -CO-O-, -O-CO-O- unterbrochen sein kann,
- n =: 1, 2, 3 oder 4 ist,
- m =: 1 oder 2 ist,
- X =: entfällt oder ein C₁-C₁₀-Rest ist, der durch -O-, -S-, -CO-O-, -O-CO-NH- oder -CO-NR¹- unterbrochen sein kann, wobei R¹ H oder C₁-C₆-Alkyl, vorzugsweise H, CH₃ oder C₂H₅ ist, und
- PG =: eine radikalisch polymerisierbare Gruppe ist, die aus Vinyl-, Allyl-, CH₂=CR²-CO-Y-, R³O-CO-C(=CH₂)-CH₂-Y- ausgewählt ist, wobei Y O oder NR⁴ ist oder entfällt, R² H oder CH₃ ist und R³ und R⁴ unabhängig voneinander jeweils H oder ein C₁-C₇-Alkyl sind.

Die Gruppe A ist ein aliphatische Gruppe, die m-fach durch den Rest [(PG)ₙ-X] bzw. n-fach durch PG, wenn X entfällt, und einfach durch die β-Ketophosphonsäuregruppe substituiert ist. Die Formel erfasst nur solche Verbindungen, die mit der chemischen Valenzlehre vereinbar sind. Wenn also A beispielsweise nur ein Kohlenstoffatom enthält, kann dieses Kohlestoffatom maximal vier Substituenten tragen.

Der Hinweis, dass ein Rest durch Heteroatome oder funktionelle Gruppen unterbrochen ist, ist so zu verstehen, dass die Heteroatome oder funktionellen Gruppen in die Kohlenstoffkette eingeschoben werden und beidseitig von C-Atomen begrenzt sind. Die Aneinanderreihung von Heteroatomen und/oder funktionellen Gruppen fällt nicht unter diese Definition.

Vorzugsweise ist A nicht oder durch 1 bis 4, insbesondere 1 bis 2 Heteroatome oder funktionelle Gruppen unterbrochen, besonders bevorzugt durch 1 oder 2 O-Atome.

X ist eine aliphatische Gruppe, die n-fach durch PG substituiert ist und die über eine weitere Bindung an A gebunden ist. X ist vorzugsweise eine C₁-C₁₀-Alkylengruppe, insbesondere eine lineare Alkylengruppe. X kann durch -O-, -S-, -CO-O-, -O-CO-NH- oder -CO-NR¹- unterbrochen sein, wobei X vorzugsweise nicht durch Heteroatome oder funktionelle Gruppen unterbrochen wird.

Bevorzugte Bedeutungen von PG sind (Meth)acryloyloxygruppen (CH₂=CR²-CO-Y- mit Y = O), insbesondere (Meth)acryloylamino-gruppen (CH₂=CR²-CO-Y- mit Y = NR⁴) und R³O-CO-C(=CH₂) -CH₂-Y-(mit Y = vorzugsweise O); R² ist jeweils H oder CH₃, R³ ist CH₃ oder C₂H₅ und R⁴ ist H, CH₃ oder C₂H₅.

Die oben genannten bevorzugten Definitionen der Variablen können unabhängig voneinander gewählt werden. Erfindungsgemäß sind naturgemäß aber solche Verbindungen besonders bevorzugt, in denen alle Variablen eine der bevorzugten und insbesondere eine der besonders bevorzugten Definitionen haben.

Bevorzugt sind somit Verbindungen, in denen die Variablen wie folgt definiert sind:
- A =: ein aliphatischer C₁-C₁₅-Rest, der durch -O- unterbrochen sein kann, vorzugsweise ein linearer C₁-C₁₀-Rest oder C₂-C₁₀-Rest, der durch 1 oder 2 O-Atome unterbrochen sein kann,
- n =: 1 oder 2, vorzugsweise 1,
- m =: 1 oder 2,
- X =: ein C₁-C₄-Alkylenrest oder entfällt, vorzugsweise ein C₁-oder C₂-Rest oder entfällt, und
- PG =: Vinyl-, Allyl-, CH₂=CR²-CO-Y- oder R³O-CO-C(=CH₂) -CH₂-Y-, wobei Y O oder NR⁴ ist oder entfällt, R² H oder CH₃ ist und R³ und R⁴ unabhängig voneinander jeweils H oder ein C₁-C₇-Alkyl sind; besonders bevorzugt CH₂=CR²-CO-Y-, mit Y = O oder NR⁴, oder R³O-CO-C(=CH₂) -CH₂-Y- mit Y = O; R² = H oder CH₃, R³ = CH₃ oder C₂H₅ und R⁴ = H, CH₃ oder C₂H₅.

Die polymerisationsfähigen β-Ketophosphonsäuren der allgemeinen Formel I können einfach hergestellt werden. Beispielsweise lassen sich OH-funktionalisierte β-Ketophosphonate mit COOH-Alkyl-funktionalisierten polymerisationsfähigen Resten zu den entsprechenden polymerisationsfähigen β-Ketophosphonaten und anschließend durch Silylierung mit z.B. Trimethylsilylbromid (TMSiBr) und Methanolyse zu den polymerisationsfähigen β-Ketophosphonsäuren der allgemeinen Formel I umsetzen:

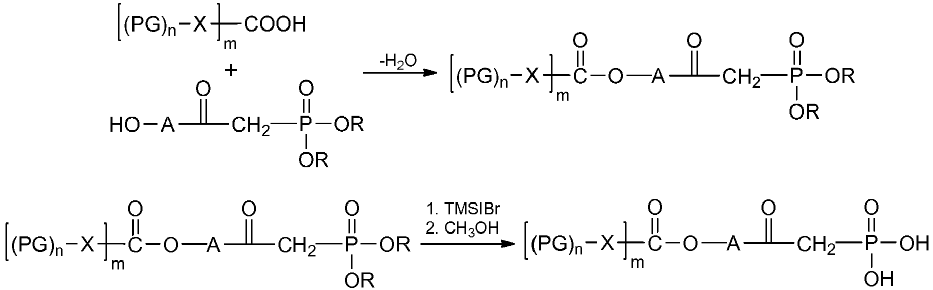

Konkret kann z.B. Methacrylsäure-3-carboxypropylester mit 5-Hydroxy-2-oxopentylphosphonsäurediethylester umgesetzt werden mit anschließender Freisetzung der Phosphonsäuregruppen:

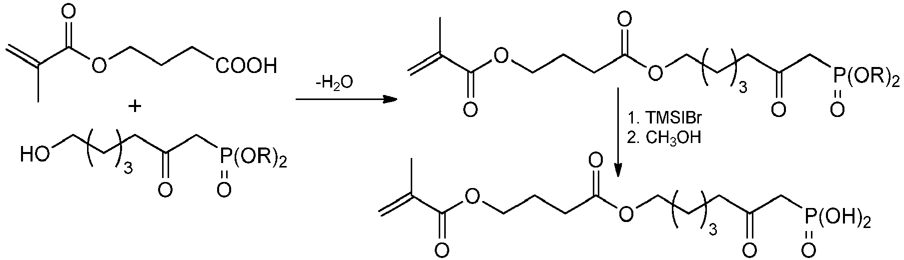

Dabei lassen sich Ketophosphonate nach der Arbuzov-Reaktion durch Umsetzung von α-Halogen-Ketonen mit Trialkylphosphiten (vgl. z.B. A. K. Bhattacharya, G. Thyagarajan, Chem. Rev. 81 (1981) 415-430), durch Acylierung von Alkylphosphonaten mit Carbonsäurederivaten in Gegenwart einer stöchiometrischen Menge an Organometallreagenz (vgl. z.B. K. M. Maloney, J. Y. L. Chung, J. Org. Chem. 74 (2009) 7554-7576), durch Oxidation von β-Hydroxyalkylphosphonaten (vgl. z.B. Koprowski et al., Tetrahedron 65 (2009) 4017-4024) und durch direkte oder katalytische Oxyphosphorylierung von Alkenen mit O₂ und H-Phosphonaten (W. Wie, J.-X. Ji, Angew. Chem. 123 (2011) 9263-9265) herstellen.

Bevorzugte Beispiele für die erfindungsgemäßen polymerisationsfähigen β-Ketophosphonsäuren der allgemeinen Formel I sind:

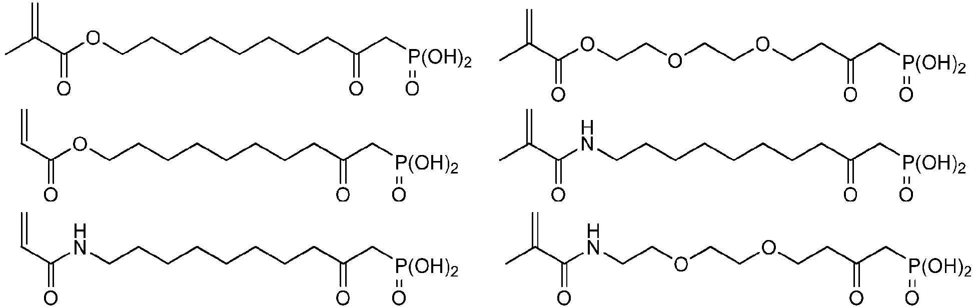

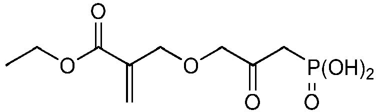

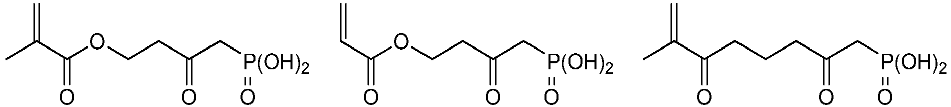

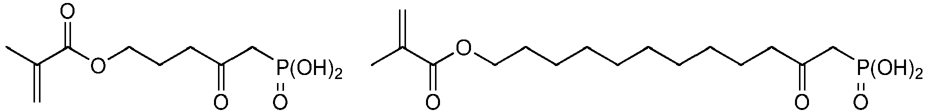

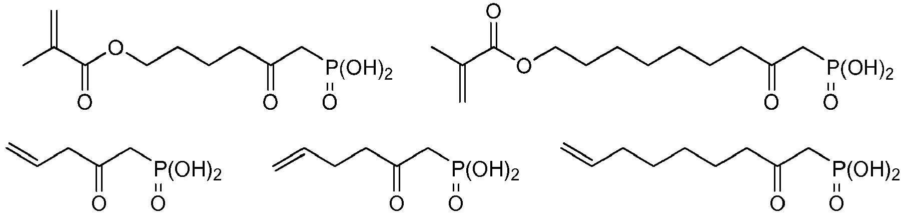

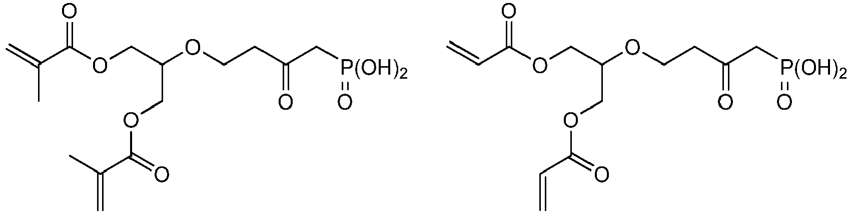

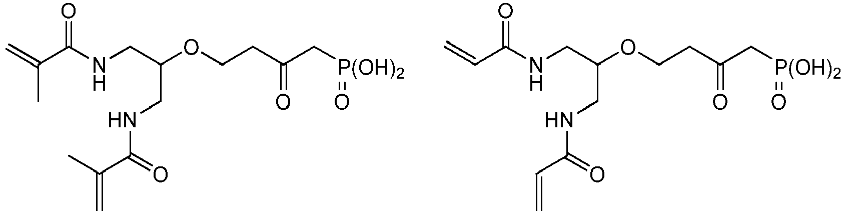

Die polymerisationsfähigen β-Ketophosphonsäuren der allgemeinen Formel I eignen sich besonders zur Herstellung von Dentalwerkstoffen, insbesondere von Dentalwerkstoffen mit selbstätzenden Eigenschaften. Sie sind sehr gut in Alkoholen, wie z.B. Ethanol und Isopropanol, und in Aceton oder in wässrigen Mischungen davon löslich. Im Vergleich zu bekannten polymerisationsfähigen Alkylphosphonsäuren mit vergleichbarer Spacerlänge zeigen z.B. die wässrig-alkoholischen Lösungen überraschenderweise einen deutlich geringeren pH-Wert, d.h. sie sind deutlich acider, was im Hinblick auf die selbstätzenden Eigenschaften vorteilhaft ist. Außerdem zeigen sie im Vergleich mit herkömmlichen Alkylphosphonsäuren eine höhere Schmelzhaftung. Ein weiterer Vorteil ist ihre gegenüber acide Phosphaten verbesserte Hydrolysestabilität. Die erfindungsgemäßen β-Ketophosphonsäuren kombinieren damit die hohe Hydrolysestabilität von Phosphonsäuren mit der hohen Acidität von Phosphorsäureestern.

Die β-Ketophosphonsäuren der Formel I werden vorzugsweise in einer Menge von 0,1 bis 50 Gew.-%, besonders bevorzugt 1 bis 40 Gew.-% und ganz besonders bevorzugt in einer Menge von 2 bis 30 Gew.-% bezogen auf die Gesamtmasse des Dentalwerkstoffs eingesetzt.

Die erfindungsgemäßen Dentalwerkstoffe auf der Basis der polymerisationsfähigen β-Ketophosphonsäuren der allgemeinen Formel I, können vorzugsweise weitere radikalisch polymerisierbare Monomere (Comonomere), besonders bevorzugt mono- oder polyfunktionelle (Meth)acrylsäurederivate enthalten. Unter monofunktionellen Monomeren werden Monomere mit einer, unter polyfunktionellen Monomeren Monomere mit zwei oder mehr, vorzugsweise zwei bis vier radikalisch polymerisierbaren Gruppen verstanden. Diesbezügliche Beispiele sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), Di-, Tri- oder Tetraethylenglycoldi(meth)-acrylat, Trimethylolpropantri(meth)acrylat, Pentaerythrit-tetra(meth)acrylat, sowie Glycerindi(meth)acrylat, 1,4-Butan-dioldi(meth)acrylat, 1,10-Decandioldi(meth)acrylat oder 1,12-Dodecandioldi(meth)acrylat.

Weiter bevorzugte Comonomere sind N-mono- oder -disubstitiuierte Acrylamide, wie z.B. N-Ethylacrylamid, N,N-Dimethylacrylamid, N-(2-Hydroxyethyl)acrylamid oder N-Methyl-N-(2-hydroxyethyl)acrylamid, bzw. N-monosubstituierte Methacrylamide, wie z.B. N-Ethylmethacrylamid oder N-(2-Hydroxyethyl)methacrylamid sowie N-Vinylpyrrolidon oder Allylether. Diese Monomere zeichnen sich durch eine hohe Hydrolysestabilität sowie eine relativ geringe Viskosität aus und eignen sich daher beispielsweise als Verdünnermonomere.

Ebenso bevorzugte Comonomere sind vernetzende Pyrrolidone, wie z.B. 1,6-Bis(3-vinyl-2-pyrrolidonyl)-hexan, oder kommerziell zugängliche Bisacrylamide, wie Methylen- oder Ethylenbisacrylamid, bzw. Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können. Auch diese Monomere zeichnen sich durch eine hohe Hydrolysestabilität aus. Sie enthalten zwei oder mehr radikalisch polymerisierbare Gruppen und eignen sich daher z.B. als Vernetzermonomere.

Vorzugweise werden als Comonomere Mischungen aus monofunktionellen und vernetzenden Monomeren verwendet, wobei Monomermischungen, die 2-Hydroxyethylmethacrylat in Kombination mit BisGMA, UDMA, Triethylenglycoldimethacrylat und/oder Decandioldimethacrylat enthalten, besonders vorteilhaft sind.

Schließlich lassen sich auch Mischungen von einem oder mehreren der voranstehend genannten Monomeren mit weiteren radikalisch polymerisierbaren, säuregruppenhaltigen Haftmonomeren verwenden. Geeignete säuregruppenhaltige Monomere sind polymerisationsfähige Carbonsäuren, wie Maleinsäure, Acrylsäure, Methacrylsäure, 2-(Hydroxymethyl)acrylsäure, 4-(Meth)acryloyl-oxyethyltrimellitsäureanhydrid, 10-Methacryloyloxydecylmalonsäure, N-(2-Hydroxy-3-methacryloyloxypropyl)-N-phenylglycin bzw. 4-Vinylbenzoesäure. Beispiele für geeignete Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[4-(Dihydroxyphosphoryl)-2-oxa-butyl]-acrylsäureethyl- oder -2,4,6-trimethyl-phenylester. Beispiele für geeignete acide polymerisationsfähige Phosphorsäureester sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat. Beispiele für geeignete polymerisationsfähige Sulfonsäuren sind Vinylsulfonsäure, 4-Vinylphenylsulfonsäure oder 3-(Methacrylamido)propylsulfonsäure. Die Gesamtmenge an weiteren säuregruppenhaltigen Monomeren wird vorzugsweise so gewählt, dass sie die Menge an Ketophosphonsäure (n) der Formel I nicht übersteigt und besonders bevorzugt darunter liegt.

Zur Initiierung der radikalischen Polymerisation enthalten die erfindungsgemäßen Dentalwerkstoffe einen Initiator für die radikalische Polymerisation. Für die Photopolymerisation werden vorzugsweise Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil, eingesetzt. Bevorzugt werden Campherchinon und 2,2-Dimethoxy-2-phenyl-acetophenon und besonders bevorzugt α-Diketone in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin, verwendet. Besonders geeignet sind auch Norrish-Typ-I-Photoinitiatoren, vor allem Acyl- oder Bisacylphosphinoxide, Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium. Dabei lassen sich auch Mischungen der verschiedenen Photoinitiatoren einsetzen, wie z.B. Dibenzoyldiethylgermanium in Kombination mit Campherchinon und 4-Dimethylaminobenzoesäureethylester.

Als Initiatoren für eine bei Raumtemperatur durchgeführte Polymerisation werden Redox-Initiatorkombinationen, wie z.B. Kombinationen von Benzoylperoxid mit N,N-Dimethyl-sym.-xylidin, N,N-Dimethyl-p-toluidin, N,N-Diethyl-3,5-di-tert-Butylanilin oder N,N-Diethanol-p-toluidin, verwendet. Darüber hinaus sind auch Redoxsysteme bestehend aus Peroxiden bzw Hydroperoxiden und Reduktionsmitteln, wie z.B. Ascorbinsäure, Barbiturate, Thioharnstoffe oder Sulfinsäuren, besonders geeignet.

Die erfindungsgemäßen Dentalwerkstoffe enthalten vorzugsweise einen Photoinitiator oder eine Kombination aus einem Photoinitiator und einem Redoxinitiator, vorzugweise einem Peroxid. Eine besonders vorteilhafte Initiatorkombination für die duale Härtung ist eine Mischung aus Campherchinon und Benzoylperoxid, wobei auch diese Initiatoren bevorzugt mit einem Amin kombiniert werden.

Weiterhin enthalten die erfindungsgemäß eingesetzten Zusammensetzungen zur Verbesserung der mechanischen Eigenschaften oder zur Einstellung der Viskosität vorzugsweise organische oder anorganische Füllstoffpartikel. Füllstoffe zur Anpassung der mechanischen Eigenschaften haben vorzugsweise einen mittleren Partikeldurchmesser von 10 nm bis 10 µm, bevorzugt von 10 nm bis 1,0 µm, Füllstoffe zur Einstellung der Viskosität vorzugsweise von 10 bis 1000 nm, bevorzugt von 10 bis 200 nm. Diese Füllstoffarten werden vorzugsweise gemeinsam eingesetzt. Wenn nicht anders angegeben, handelt es sich bei dem mittleren Partikeldurchmesser um den massengemittelten Durchschnittswert.

Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO₂, nanopartikuläre oder mikrofeine Füllstoffe, wie pyrogene Kieselsäure oder Fällungskieselsäure sowie Minifüllstoffe, wie Quarz-, Glaskeramik- oder Glaspulver mit einem mittleren Partikeldurchmesser von 0,01 bis 1 µm sowie röntgenopake Füllstoffe, wie Ytterbiumtrifluorid oder nanopartikuläres Tantal(V)-oxid bzw. Bariumsulfat. Bevorzugte organische Füllstoffe sind Füllstoffe auf der Basis von Poly(meth)acrylaten, wie z.B. PMMA, oder Cellulosederivaten, wie z.B. Carboxymethylcellulose, die nach dem Härten auf die oben genannte Partikelgröße gemahlen werden. Die organischen Füllstoffe können ihrerseits mit den genannten anorganischen Füllstoffen gefüllt sein.

Lösungsmittelhaltige Dentalwerkstoffe stellen eine weitere bevorzugte Ausführungsform der Erfindung dar. Hierbei kommen besonders Wasser und polare organische Lösungsmittel wie Aceton, Isopropanol und insbesondere Ethanol sowie Mischungen dieser Lösungsmittel in Betracht. Besonders bevorzugt sind Mischungen aus Wasser und polaren organischen Lösungsmitteln, insbesondere Mischungen aus Wasser und Ethanol, Wasser und Aceton oder Wasser, Ethanol und Aceton.

Gegebenenfalls können die erfindungsgemäß eingesetzten Zusammensetzungen weitere Additive enthalten wie z.B. Stabilisatoren, Aromastoffe, Farbmittel, mikrobizide Wirkstoffe, Fluoridionen abgebende Additive, optische Aufheller, Weichmacher und/oder UV-Absorber.

Dabei sind erfindungsgemäße Dentalmaterialien bevorzugt, welche die folgenden Komponenten enthalten:
a) 0,1 bis 50 Gew.-%, bevorzugt 1 bis 40 Gew.-% und besonders bevorzugt 2 bis 30 Gew.-% an polymerisationsfähigen β-Ketophosphonsäuren der allgemeinen Formel I,
b) 0,01 bis 10 Gew.-%, besonders bevorzugt 0,1 bis 3,0 Gew.-% an Initiator,
c) 0 bis 80 Gew.-%, bevorzugt 0 bis 60 Gew.-% und besonders bevorzugt 5 bis 50 Gew.-% an zusätzlichen Monomeren,
d) 0 bis 80 Gew.-% Füllstoff,
e) 0 bis 70 Gew.-%, bevorzugt 0 bis 60 Gew.-% und besonders bevorzugt 0 bis 50 Gew.-% Lösungsmittel, und ggf.
f) 0,01 bis 10 Gew.-%, bevorzugt 0,01 bis 3 Gew.-% weitere Additive.

Als zusätzlichen Monomere (c) enthalten die erfindungsgemäßen Dentalwerkstoffe vorzugweise 5 bis 40 Gew.-% an nichtaciden mono- oder multifunktionellen Monomeren und/oder 0 bis 60 Gew.-%, bevorzugt 0 bis 30 Gew.-% an aciden Monomeren.

Die Menge des oder der Füllstoffe (d) richtet sich nach dem gewünschten Anwendungszweck. Dentalwerkstoffe zur Verwendung als Adhäsive enthalten vorzugsweise 0 bis 20 Gew.-% und Dentalwerkstoffe zur Anwendung als Zement oder Füllungsmaterial (Komposit) vorzugweise 20 bis 80 Gew.-% Füllstoff. Dentalwerkstoffe zur Anwendung als Zement oder Füllungsmaterial enthalten vorzugweise kein Lösungsmittel.

Dentalwerkstoffe zur Anwendung als Adhäsive weisen vorzugsweise die folgende Zusammensetzung auf:
a) 0,1 bis 50 Gew.-%, bevorzugt 1 bis 40 Gew.-% und besonders bevorzugt 2 bis 30 Gew.-% an polymerisationsfähigen β-Ketophosphonsäuren der allgemeinen Formel I,
b) 0,01 bis 10 Gew.-%, besonders bevorzugt 0,1 bis 3,0 Gew.-% an Initiator,
c) 0 bis 60 Gew.-%, bevorzugt 0 bis 40 Gew.-% und besonders bevorzugt 5 bis 40 Gew.-% an nichtaciden mono- oder multifunktionellen Monomeren und/oder 0 bis 60 Gew.-% und bevorzugt 0 bis 30 Gew.-% an aciden Comonomeren,
d) 0 bis 20 Gew.-% (Adhäsiv) an Füllstoffen,
e) 0 bis 70 Gew.-%, bevorzugt 0 bis 60 Gew.-% und besonders bevorzugt 0 bis 50 Gew.-% Lösungsmittel, vorzugsweise Wasser oder eine Mischung von Wasser, Ethanol und/oder Aceton, und ggf.
f) 0,01 bis 3 % weitere Additive.

Alle Prozentangaben beziehen sich jeweils auf die Gesamtmasse der Zusammensetzung.

Besonders bevorzugt sind solche Dentalwerkstoffe, welche aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind.

Die Dentalwerkstoffe eigenen sich besonders als Adhäsive, Zemente, Füllungsmaterialen oder Beschichtungsmaterialien. Die Dentalwerkstoffe eignen sich primär zur intraoralen Anwendung durch den Zahnarzt zur Restauration geschädigter Zähne (klinische Materialien). Sie können aber auch extraoral eingesetzt werden, beispielsweise bei der Herstellung oder Reparatur von Dentalrestaurationen (technische Materialien).

Die Erfindung wird im Folgenden anhand von Beispielen näher erläutert.

### Ausführungsbeispiele:

### Beispiel 1:

### Synthese von 9-Methacryloyloxy-2-oxononylphosphonsäure (MOPA)

### a) Synthese von 2-Oxo-9-THP-oxynonylphosphonsäure-diethylester 1

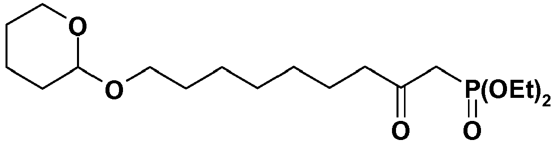

Eine Lösung von käuflichen Diethyl-(2-oxopropyl)phosphonat (10.0 g, 51.5 mmol, 1.1 Äquivalente), das analog der Literatur (Kosobokov, M. D.; Titanyuk, I. D.; Beletskaya, I. P. Mendeleev Communications 2011, 21, 142-143) hergestellt wurde, in trockenem THF (15 ml) wurde tropfenweise zu einer gerührten Mischung von NaH (2.27 g, 56.7 mmol, 1.2 Äquivalente), einer 60 % igen Dispersion in Mineralöl, die mit Hexan (2 x 20 ml) vorgewaschen wurde) in trockenem THF (20 ml) bei 0 °C zugetropft. Die Reaktionsmischung wurde auf Raumtemperatur erwärmt, 1 h gerührt, dann wieder auf 0 °C abgekühlt, eine Lösung von n-BuLi (22.5 ml einer 2.5 M Lösung in Hexan, 56.2 mmol, 1.2 Äquivalente.) zugetropft und die Lösung 30 Min bei 0°C gerührt. Dann wurde eine Lösung von 2-(6-Bromhexyl-oxy)tetrahydro-2H-pyran (12.4 g, 46.8 mmol), das analog der Literatur (Fu, Y.; Weng, Y.; Wen-Xu, H.; Qinghai, Z. Synlett 2011, 6, 809-812) hergestellt wurde, in trockenem THF (15 ml) zugegeben, die Reaktionsmischung auf Raumtemperatur erwärmt und 15 h gerührt. Anschließend wurde das Reaktionsprodukt vorsichtig in eisgekühlte wässrige NH₄Cl-Lösung (150 ml) gegeben und die wässrige Phase mit Diethylether extrahiert (3 x 100 ml). Die kombinierten organischen Phasen wurden über wasserfreiem Na₂SO₄ getrocknet und im Vakuum eingeengt. Das erhaltene Rohprodukt wurde durch Flash-Säulenchromatographie (Eluent: Ethylacetat/Hexan: 75/25) gereinigt und ergab 9.6 g (25.4 mmol) des Phosphonates **1** als schwach gelbliche Flüssigkeit. Ausbeute: 54 %.
¹H NMR (400 MHz, CDCl₃) : δ = 1.23-1.40 (m, 6H, CH₂) ; 1.35 (t, ³J_{HH} = 7.0 Hz, 6H, POCH₂CH₃) ; 1.46-1.63 (m, 8H, CH₂) ; 1.65-1.76 (m, 1H, CH₂) ; 1.77-1.89 (m, 1H, CH₂); 2.61 (t, ³J_{HH} = 7.3 Hz, 2H, CH₂CH₂C=O) ; 3.07 (d, ²J_{HP} = 22.8 Hz, 2H, CH₂P) ; 3.37 (dt, ²J_{HH} = 9.6 Hz, ³J_{HH} = 6.5 Hz, 1H, CH₂O); 3.46-3.54 (m, 1H, CH₂O); 3.72 (dt, ²J_{HH} = 9.6 Hz, ³J_{HH} = 6.9 Hz, 1H, CH₂O); 3.83-3.90 (m, 1H, CH₂O); 4.09-4.20 (m, 4H, POCH₂CH₃); 4.54-4.59 (m, 1H, OCHO).
³¹P NMR (162 MHz, CDCl₃) : 20.0.
¹³C NMR (101 MHz, CDCl₃) : δ = 16.3 (d, ³J_{CP} = 6.3 Hz, POCH₂CH₃) ; 19.7 (CH₂); 23.3 (CH₂); 25.5 (CH₂); 26.0 (CH₂); 28.9 (CH₂); 29.2 (CH₂); 29.7 (CH₂); 30.8 (CH₂); 42.4 (d, ¹J_{CP} = 127.3 Hz, CH₂P); 44.0 (CH₂CH₂C=O) ; 62.3 (CH₂O) ; 62.4 (d, ²J_{CP} = 6.4 Hz, POCH₂CH₃) ; 67.5 (CH₂O) ; 98.8 (OCHO); 202.1 (d, ²J_{CP} = 6.0 Hz, C=O).

### b) 9-Hydroxy-2-oxononylphosphonsäure-diethylester 2

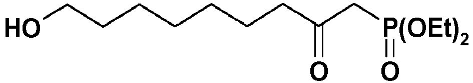

Das Phosphonat **1** (47.2 mmol) und Pyridinium-toluensulfonat (1.19 g, 4.72 mmol) wurden zu Ethanol gegeben (500 ml), die Mischung bei 55 °C für 3 h gerührt und dann im Vakuum eingeengt. Das erhaltene Rohprodukt wurde durch Flash-Säulenchromatographie (Eluent: Ethylacetat/Hexan: 95/5) gereinigt und ergab 11.04 g (37.6 mmol) des Phosphonates **2** als schwach gelbliche Flüssigkeit. Ausbeute: 80 %.
¹H NMR (400 MHz, CDCl₃) : δ = 1.26-1.40 (m, 6H, CH₂) ; 1.34 (t, ³J_{HH} = 6.9 Hz, 6H, POCH₂CH₃); 1.51-1.64 (m, 4H, CH₂) ; 2.62 (t, ³J_{HH} = 7.4 Hz, 2H, CH₂CH₂C=O) ; 3.07 (d, ²J_{HP} = 22.8 Hz, 2H, CH₂P); 3.63 (t, ³J_{HH} = 6.6 Hz, 2H, CH₂OH) ; 4.09-4.20 (m, 4H, POCH₂CH₃).
³¹P NMR (162 MHz, CDCl₃) : 20.0.
¹³C NMR (101 MHz, CDCl₃) : δ = 16.2 (d, ³J_{CP} = 6.2 Hz, POCH₂CH₃) ; 23.2 (CH₂) ; 25.5 (CH₂) ; 28.8 (CH₂) ; 29.0 (CH₂) ; 32.7 (CH₂) ; 42.3 (d, ¹J_{CP} = 127.3 Hz, CH₂P) ; 43.9 (CH₂CH₂C=0) ; 62.5 (d, ²J_{CP} = 6.5 Hz, POCH₂CH₃) ; 62.7 (CH₂OH) ; 202.1 (d, ²J_{CP} = 6.2 Hz, C=O).

### c) 9-Methacryloyloxy-2-oxononylphosphonsäurediethylester 3

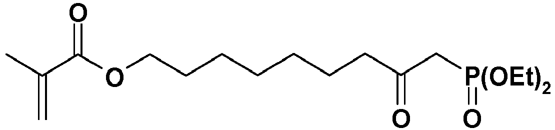

Methacrylsäureanhydrid (6.14 ml, 41.2 mmol, 1.1 Äquivalente) wurde unter Rühren tropfenweise zu einer Lösung des Hydroxyphosphonates **2** (37.5 mmol), Triethylamin (5.75 ml, 41.2 mmol, 1.1 Äquivalente.) und 4-Dimethylaminopyridin (229 mg, 1.9 mmol, 5 mol %) in wasserfreiem Methylenchlorid (100 ml) gegeben. Nach 15 h Rühren wurde die Reaktionsmischung mit destilliertem Wasser (100 ml) gewaschen, die organische Phase abgetrennt, über wasserfreiem Na₂SO₄ getrocknet und im Vakuum eingeengt. Das erhaltene Rohprodukt wurde durch Flash-Säulenchromatographie (Eluent: Ethylacetat/Hexan: 80/20) gereinigt und ergab 11.53 g (31.9 mmol) des Phosphonates **3** als schwach gelbliche Flüssigkeit. Ausbeute: 85 %.
¹H NMR (400 MHz, CDCl₃) : δ = 1.23-1.42 (m, 6H, CH₂) ; 1.32 (t, ³J_{HH} = 7.1 Hz, 6H, POCH₂CH₃) ; 1.52-1.70 (m, 4H, CH₂) ; 1.93 (ls, 3H, CH₃) ; 2.61 (t, ³J_{HH} = 7.3 Hz, 2H, CH₂CH₂C=O) ; 3.05 (d, ²J_{HP} = 22.9 Hz, 2H, CH₂P); 4.08-4.19 (m, 6H, POCH₂CH₃ and CH₂OC=O); 5.51-5.55 (m, 1H, CH₂=C); 6.08 (ls, 1H, CH₂=C).
³¹P NMR (162 MHz, CDCl₃) : 20.0.
¹³C NMR (101 MHz, CDCl₃) : δ = 16.3 (d, ³J_{CP} = 6.3 Hz, POCH₂CH₃); 18.3 (CH₃) ; 23.3 (CH₂); 25.8 (CH₂); 28.5 (CH₂); 28.8 (CH₂); 29.0 (CH₂); 42.4 (d, ¹J_{CP} = 127.3 Hz, CH₂P); 44.0 (CH₂CH₂C=O); 62.5 (d, ²J_{CP} = 6.4 Hz, POCH₂CH₃) ; 64.7 (CH₂OC=O) ; 125.1 (CH₂=C) ; 136.5 (CH₂=C); 167.5 (OC=O); 202.0 (d, ²J_{CP} = 6.2 Hz, PCH₂C=O).

### d) 9-Methacryloyloxy-2-oxononylphosphonsäure (MOPA)

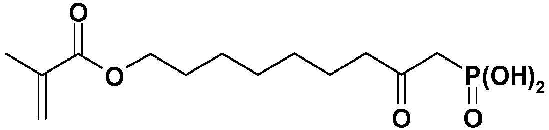

Trimethylsilylbromid (12.5 ml, 94.7 mmol, 3.0 eq.) wurde zu einer Lösung des Phosphonates **3** (11.43 g, 31.6 mmol) in wasserfreiem Methylenchlorid (100 ml) gegeben und 5 h bei 30 °C gerührt. Danach wurde das Reaktionsprodukt im Vakuum eingeengt, Methanol (100 ml) zugegeben und 30 Min bei Raumtemperatur gerührt. Nach Zugabe von BHT (250 ppm) wurde die Lösung im Feinvakuum bis zur Gewichtskonstanz eingeengt und ergab 9.6 g (31.4 mmol) der Ketophosphonsäure MOPA als schwach gelbliche Flüssigkeit. Ausbeute: 99 %.
¹H NMR (400 MHz, CDCl₃) : δ = 1.23-1.42 (m, 6H, CH₂) ; 1.52-1.71 (m, 4H, CH₂) ; 1.94 (ls, 3H, CH₃) ; 2.63 (t, ³J_{HH} = 7.3 Hz, 2H, CH₂CH₂C=O) ; 3.19 (d, ²J_{HP} = 22.7 Hz, 2H, CH₂P) ; 4.13 (t, ³J_{HH} = 6.6 Hz, 2H, CH₂OC=0) ; 5.54-5.58 (m, 1H, CH₂=C); 6.09 (ls, 1H, CH₂=C); 10.25 (ls, 2H, POH).
³¹P NMR (162 MHz, CDCl₃) : 22.2.
¹³C NMR (101 MHz, CDCl₃) : δ = 18.3 (CH₃) ; 23.2 (CH₂); 25.7 (CH₂); 28.5 (CH₂); 28.7 (CH₂); 28.9 (CH₂); 42.3 (d, ¹J_{CP} = 131.3 Hz, CH₂P); 44.2 (CH₂CH₂C=O) ; 64.9 (CH₂OC=O) ; 125.5 (CH₂=C) ; 136.4 (CH₂=C) ; 167.8 (OC=O) ; 204.6 (d, ²J_{CP} = 6.4 Hz, PCH₂C=O).

### Beispiel 2:

### Untersuchung der Photopolymerisation von 9-Methacryloyloxy-2-oxononylphosphonsäure MOPA mittels DSC

Zu einer Mischung aus dem Vernetzer N,N'-Diethyl-1,3-bis(acrylamido)-propan (DEPBA) und MOPA im Molverhältnis 8:2 wurde 0.1 Gew.-% des Photoinitiators Bis(4-methoxybenzoyl)diethylgermanium zugesetzt. Die Mischung wurde in einem Differential-Scanning-Kalorimeter (Diamond, Fa. Perkin Elmer) mit Photopolymerisationsaufsatz durch Bestrahlung mit einer LED-Lampe (Bluephase, Fa. Ivoclar Vivadent) für 2 Minuten bei 37 °C polymerisiert. Es ergab sich eine im Vergleich zu dem reinen Vernetzer ähnlich hohe maximale Polymerisationsgeschwindigkeit (0.078 s⁻¹) und übereinstimmender Doppelbindungsumsatz (63%) der Mischung

### Beispiel 3:

### Bestimmung des pH-Werts einer MOPA-Lösung

Es wurde der pH-Wert von 20 %-igen Lösungen von MOPA, 10-(Methacryloyloxy)decyldihydrogenphosphat (MDP) und 10-(Methacryloyloxy)decylphosphonsäure (MDPA) in einer Mischung im Gewichtsverhältnis 1:1 aus Wasser und Ethanol bestimmt. Für MOPA ergab sich ein pH-Wert von 1.9, während für das Dihydrogenphosphat MDP ein pH-Wert von 1.6 und für das Phosphonsäuremonomer MDPA ein pH-Wert von 2.3 bestimmt wurde. Damit zeigt sich überraschenderweise, dass die untersuchte Ketophosphonsäure deutlich stärker sauer als eine Alkylphosphonsäure mit ähnlicher C-Anzahl der Spacergruppe ist.

### Beispiel 4:

### Adhäsive und Haftuntersuchungen auf der Basis der 9-Methacry-loyloxy-2-oxononylphosphonsäure MOPA

Zur Untersuchung der Dentin- und Schmelzhaftung auf Rinderzähne wurden Adhäsive mit der in Tabelle 1 angegebenen Zusammensetzung hergestellt. Rinderzähne wurden so in Kunststoffzylinder eingebettet, dass sich das Dentin bzw. der Schmelz und der Kunststoff in einer Ebene befanden. Mit einem Microbrush wurde eine Schicht Adhäsiv obiger Zusammensetzung aufgepinselt, ca. 20 s das Adhäsiv auf der Zahnhartsubstanz bewegt, mit einem Luftgebläse zur Entfernung des Lösungsmittels kurz verblasen und für 10 s mit einer LED-Lampe (Bluephase, Ivoclar Vivadent) belichtet. Auf die Adhäsivschicht polymerisierte man einen Kompositzylinder aus Tetric® EvoCeram (Ivoclar Vivadent) auf.

Anschließend werden die Prüfkörper 24 h bei 37 °C in Wasser gelagert und die Scherhaftfestigkeit entsprechend der ISO-Richtlinie "ISO 2003-ISO TR 11405: Dental Materials Guidance on Testing of Adhesion to Tooth Structure" bestimmt: Adhäsiv A: Dentin: 34.4 MPa und Schmelz 30.3 MPa; Adhäsiv B: 22.6 MPa und Schmelz 16.8 MPa.

Die Ergebnisse belegen, dass Schmelz-/Dentin-Adhäsive auf der Basis der polymerisationsfähigen β-Ketophosphonsäuren hohe Schmelz- und Dentinhaftwerte mit dentalen Kompositen ergeben.

**Tabelle 1**

| **Zusammensetzung der Adhäsive (Angaben in Masse-%)** | | |
|---|---|---|
| **Komponente** | **Adhäsiv A** | **Adhäsiv B (Vergleich)** |
| MOPA | 15.0 | - |
| MDPA | - | 15.0 |
| Bis-GMA¹⁾ | 19.0 | 19.0 |
| DEPBA | 43.2 | 43.2 |
| Aerosil R709²⁾ | 1.4 | 1.4 |
| Photoinitiator³⁾ | 2.6 | 2.6 |
| deionisiertes Wasser | 14.6 | 14.6 |
| Isopropanol | 4.2 | 4.2 |

| | | |
|---|---|---|
| ¹⁾ Additionsprodukt aus Methacrylsäure und Bisphenol-A-diglycidylether ²⁾ methacrylsilanisierte pyrogene Kieselsäure mit einer mittleren Primärteilchengröße von 40 nm (Degussa) ³⁾ Mischung aus Campherchinon (0.9%), 4-Dimethylbenzoesäureethylester (0.4%) und dem Acylphosphinoxid Lucerin TPO (BASF; 1.3%) | | |

## Patentansprüche

1. Dentalwerkstoff, **dadurch gekennzeichnet, dass** er mindestens eine β-Ketophosphonsäure gemäß der allgemeinen Formel I 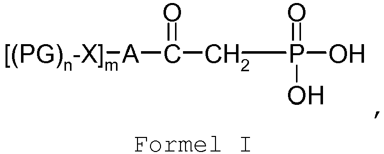 in der
A = ein aliphatischer C₁-C₁₈-Rest ist, der durch -O-, -S-, -CO-O-, -O-CO-O- unterbrochen sein kann,
n = 1, 2, 3 oder 4 ist,
m = 1 oder 2 ist,
X = entfällt oder ein C₁-C₁₀-Rest ist, der durch -O-, -S-, -CO-O-, -O-CO-NH- oder -CO-NR¹- unterbrochen sein kann, wobei R¹ H oder C₁-C₆-Alkyl ist, und
PG = Vinyl-, Allyl-, CH₂=CR²-CO-Y- oder R³O-CO-C(=CH₂) - CH₂-Y- ist, wobei Y O oder NR⁴ ist oder entfällt, R² H oder CH₃ ist und R³ und R⁴ unabhängig voneinander jeweils H oder C₁-C₇-Alkyl sind, und
mindestens einen Initiator für die radikalische Polymerisation enthält.

2. Dentalwerkstoff nach Anspruch 1, wobei
A = ein aliphatischer C₁-C₁₅-Rest ist, der durch -O-unterbrochen sein kann,
n = 1 oder 2 ist,
m = 1 oder 2 ist,
X = ein C₁-C₄-Alkylenrest ist oder entfällt, und
PG = Vinyl-, Allyl-, CH₂=CR²-CO-Y- oder R³O-CO-C(=CH₂) -CH₂-Y- ist, wobei Y O oder NR⁴ ist oder entfällt, R² H oder CH₃ ist und R³ und R⁴ unabhängig voneinander jeweils H oder C₁-C₇-Alkyl sind.

3. Dentalwerkstoff nach Anspruch 1, wobei
A = ein linearer C₁-C₁₀-Rest ist, der durch 1 oder 2 O-Atome unterbrochen sein kann,
n = 1 ist,
m = 1 oder 2 ist,
X = ein C₁- oder C₂-Rest ist oder entfällt, und
PG = Vinyl-, Allyl-, CH₂=CR²-CO-Y- oder R³O-CO-C(=CH₂) -CH₂-Y- ist, wobei Y O oder NR⁴ ist oder entfällt, R² H oder CH₃ ist und R³ und R⁴ unabhängig voneinander jeweils H oder C₁-C₇-Alkyl sind.

4. Dentalwerkstoff nach Anspruch 3, wobei
A = ein linearer aliphatischer C₂-C₁₀-Rest ist, der durch 1 oder 2 -O- unterbrochen sein kann,
n = 1 ist,
m = 1 oder 2 ist,
X = ein C₁-C₂-Rest ist oder entfällt,
PG = CH₂=CR²-CO-Y-, wobei Y O oder NR⁴ ist, oder R³O-CO-C(=CH₂)-CH₂-Y- ist, wobei Y O ist, und wobei R² H oder CH₃ ist, R³ CH₃ oder C₂H₅ und R⁴ H, CH₃ oder C₂H₅ ist.

5. Dentalwerkstoff nach einem der Ansprüche 1 bis 4, der zusätzlich mindestens ein weiteres radikalisch polymerisierbares Monomer enthält.

6. Dentalwerkstoff nach Anspruch 5, der als weiteres Monomer ein oder mehrere mono- und/oder polyfunktionelle (Meth)acrylsäurederivate und/oder (Meth)acrylamidderivate enthält.

7. Dentalwerkstoff nach einem der Ansprüche 1 bis 6, der zusätzlich mindestens ein Lösungsmittel enthält.

8. Dentalwerkstoff nach Anspruch 7, der als Lösungsmittel Wasser oder eine Mischung aus Wasser und einem polaren organischen Lösungsmittel enthält.

9. Dentalwerkstoff nach einem der Ansprüche 1 bis 8, der
a) 0,1 bis 50 Gew.-% β-Ketophosphonsäure der allgemeinen Formel I,
b) 0,01 bis 10 Gew.-% Initiator,
c) 0 bis 80 Gew.-% weiteres Monomer,
d) 0 bis 80 Gew.-% Füllstoff,
e) 0 bis 70 Gew.-% Lösungsmittel enthält.

10. Dentalwerkstoff nach Anspruch 9 zur Verwendung als Adhäsiv, der 0 bis 20 Gew.-% Füllstoff enthält.

11. Dentalwerkstoff nach Anspruch 10, der
a) 0,1 bis 50 Gew.-%, bevorzugt 1 bis 40 Gew.-% und besonders bevorzugt 2 bis 30 Gew.-% an polymerisationsfähigen β-Ketophosphonsäuren der allgemeinen Formel I,
b) 0,01 bis 10 Gew.-%, bevorzugt 0,1 bis 3,0 Gew.-% an Initiator,
c) 0 bis 60 Gew.-%, bevorzugt 0 bis 40 Gew.-% und besonders bevorzugt 5 bis 40 Gew.-% an nichtaciden mono- oder multifunktionellen Monomeren und/oder 0 bis 60 Gew.-% und bevorzugt 0 bis 30 Gew.-% an aciden Monomeren,
d) 0 bis 20 Gew.-% Füllstoff,
e) 0 bis 70 Gew.-%, bevorzugt 0 bis 60 Gew.-% und besonders bevorzugt 0 bis 50 Gew.-% Lösungsmittel enthält.

12. Dentalwerkstoff nach Anspruch 9 zur Verwendung als Zement oder Füllungsmaterial, der 20 bis 80 Gew.-% Füllstoff enthält.

13. Dentalwerkstoff nach einem der Ansprüche 1 bis 12 zur intraoralen Verwendung als Adhäsiv, Füllungsmaterial oder Zement.

14. Verwendung eines Dentalwerkstoffs nach einem der Ansprüche 1 bis 12 zur extraoralen Anfertigung oder Reparatur von Dentalrestaurationen.

## Claims

1. Dental material, **characterised in that** it comprises at least one β-ketophosphonic acid according to the general Formula I 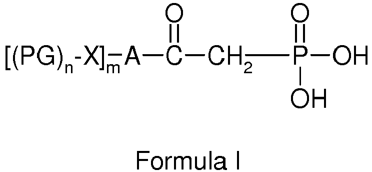 in which
A = is an aliphatic C₁-C₁₈ residue which may be interrupted by -O-, -S-, -CO-O- or -O-CO-O-,
n = is 1, 2, 3 or 4,
m = is 1 or 2,
X = is absent or a C₁-C₁₀ residue that may be interrupted by -O-, -S-, -CO-O-, -O-CO-NH- or -CO-NR¹-, wherein R¹ is H or C₁-C₆ alkyl, and
PG = vinyl, allyl, CH₂=CR²-CO-Y- or R³O-CO-C(=CH₂)-CH₂-Y-, wherein Y is O or NR⁴ or is absent, R² is H or CH₃ and R³ and R⁴ independently of each other are each H or C₁-C₇ alkyl, and
at least one initiator for free radical polymerisation.

2. Dental material according to claim 1, wherein
A = is an aliphatic C₁-C₁₅ residue which may be interrupted by -O-,
n = is 1 or 2,
m = is 1 or 2,
X = is a C₁-C₄ alkylene group or is absent, and
PG = is vinyl, allyl, CH₂=CR²-CO-Y- or R³O-CO-C(=CH₂)-CH₂-Y-, wherein Y is O or NR⁴ or is absent, R² is H or CH₃ and R³ and R⁴ independently of each other are each H or C₁-C₇ alkyl.

3. Dental material according to claim 1, wherein
A = is a linear aliphatic C₁-C₁₀ residue that may be interrupted by 1 or 2 O-atoms,
n = is 1,
m = is 1 or 2,
X = is a C₁ or C₂ group or is absent, and
PG = is vinyl, allyl, CH₂=CR²-CO-Y- or R³O-CO-C(=CH₂)-CH₂-Y-, wherein Y is O or NR⁴ or is absent, R² is H or CH₃ and R³ and R⁴ independently of each other are each H or C₁-C₇ alkyl.

4. Dental material according to claim 3, wherein
A = is a linear aliphatic C₂-C₁₀ residue that may be interrupted by 1 or 2 -O-,
n = is 1,
m = is 1 or 2,
X = is a C₁-C₂ group or is absent,
PG = is CH₂=CR²-CO-Y-, wherein Y is O or NR⁴, or is R³O-CO-C(=CH₂)-CH₂-Y-, wherein Y is O, and wherein R² is H or CH₃, R³ is CH₃ or C₂H₅ and R⁴ is H, CH₃ or C₂H₅.

5. Dental material according to one of claims 1 to 4 which additionally comprises at least one additional radically polymerisable monomer.

6. Dental material according to claim 5 which comprises one or more mono- and/or polyfunctional (meth)acrylic acid derivatives and/or (meth)acrylamide derivatives as the additional monomer.

7. Dental material according to one of claims 1 to 6 which additionally comprises at least one solvent.

8. Dental material according to claim 7 which comprises water or a mixture of water and a polar organic solvent as the solvent.

9. Dental material according to one of claims 1 to 8 which comprises
a) 0.1 to 50 wt % β-ketophosphonic acid of the general Formula I,
b) 0.01 to 10 wt % initiator,
c) 0 to 80 wt % additional monomer,
d) 0 to 80 wt % filler,
e) 0 to 70 wt % solvent.

10. Dental material according to claim 9 for use as an adhesive, which comprises 0 to 20 wt % filler.

11. Dental material according to claim 10 which comprises
a) 0.1 to 50 wt %, preferably 1 to 40 wt % and particularly preferably 2 to 30 wt % of polymerisable β-ketophosphonic acids of the general Formula I,
b) 0.01 to 10 wt %, preferably 0.1 to 3.0 wt % of initiator,
c) 0 to 60 wt %, preferably 0 to 40 wt % and particularly preferably 5 to 40 wt % of non-acidic mono- or multifunctional monomers and/or 0 to 60 wt % and preferably 0 to 30 wt % of acidic monomers,
d) 0 to 20 wt % filler,
e) 0 to 70 wt %, preferably 0 to 60 wt % and particularly preferably 0 to 50 wt % solvent.

12. Dental material according to claim 9 for use as a cement or filling material, which comprises 20 to 80 wt % filler.

13. Dental material according to one of claims 1 to 12 for intraoral use as an adhesive, filling material or cement.

14. Use of a dental material according to one of claims 1 to 12 for extra oral production or repair of dental restorations.

## Revendications

1. Matériau dentaire, **caractérisé en ce qu'**il contient au moins un acide β-cétophosphonique conforme à la formule générale I 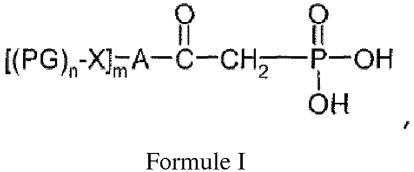 dans laquelle
A = un radical aliphatique en C₁-C₁₈ qui peut être interrompu par -O-, -S-, -CO-O-, -O-CO-O-,
n vaut 1, 2, 3 ou 4,
m vaut 1 ou 2,
X est absent ou est un radical en C₁-C₁₀ qui peut être interrompu par -O-, -S-, -CO-O-, -O-CO-NH- ou -CO-NR¹-, R¹ étant un atome d'hydrogène ou un groupe alkyle en C₁-C₆, et
PG = vinyl-, allyl-, CH₂=CR²-CO-Y- ou R³O-CO-C(=CH₂)-CH₂-Y-, Y représentant un atome d'oxygène ou NR⁴ ou étant absent, R² représentant un atome d'hydrogène ou CH₃ et R³ et R⁴ représentant chacun, indépendamment l'un de l'autre, un atome d'hydrogène ou un groupe alkyle en C₁-C₇, et
au moins un amorceur pour la polymérisation radicalaire.

2. Matériau dentaire selon la revendication 1, dans lequel
A = un radical aliphatique en C₁-C₁₅ qui peut être interrompu par -O-,
n vaut 1 ou 2,
m vaut 1 ou 2,
X est absent ou est un radical alkylène en C₁-C₄, et
PG = vinyl-, allyl-, CH₂=CR²-CO-Y- ou R³O-CO-C(=CH₂)-CH₂-Y-, Y représentant un atome d'oxygène ou NR⁴ ou étant absent, R² représentant un atome d'hydrogène ou CH₃ et R³ et R⁴ représentant chacun, indépendamment l'un de l'autre, un atome d'hydrogène ou un groupe alkyle en C₁-C₇.

3. Matériau dentaire selon la revendication 1, dans lequel
A = un radical linéaire en C₁-C₁₀ qui peut être interrompu par 1 ou 2 atomes d'oxygène,
n vaut 1,
m vaut 1 ou 2,
X est un radical en C₁ ou C₂ ou est absent, et
PG = vinyl-, allyl-, CH₂=CR²-CO-Y- ou R³O-CO-C(=CH₂)-CH₂-Y-, Y représentant un atome d'oxygène ou NR⁴ ou étant absent, R² représentant un atome d'hydrogène ou CH₃ et R³ et R⁴ représentant chacun, indépendamment l'un de l'autre, un atome d'hydrogène ou un groupe alkyle en C₁-C₇.

4. Matériau dentaire selon la revendication 3, dans lequel
A = un radical linéaire aliphatique en C₂-C₁₀ qui peut être interrompu par 1 ou 2 atomes d'oxygène,
n vaut 1,
m vaut 1 ou 2,
X est un radical en C₁ ou C₂ ou est absent, et
PG = CH₂=CR²-CO-Y-, Y représentant un atome d'oxygène ou NR⁴, ou R³O-CO-C(=CH₂)-CH₂-Y-, Y représentant un atome d'oxygène, et R² représentant un atome d'hydrogène ou CH₃, R³ représentant un groupe CH₃ ou C₂H₅ et R⁴ représentant un atome d'hydrogène ou un groupe CH₃ ou C₂H₅.

5. Matériau dentaire selon l'une quelconque des revendications 1 à 4, qui contient en outre au moins un autre monomère polymérisable par voie radicalaire.

6. Matériau dentaire selon la revendication 5, qui contient comme autre monomère un ou plusieurs dérivé(s) d'acide (méth)acrylique et/ou dérivé(s) de (méth)acrylamide, mono- et/ou polyfonctionnel(s).

7. Matériau dentaire selon l'une quelconque des revendications 1 à 6, qui contient en outre au moins un solvant.

8. Matériau dentaire selon la revendication 7, qui contient en tant que solvant de l'eau ou un mélange d'eau et d'un solvant organique polaire.

9. Matériau dentaire selon l'une quelconque des revendications 1 à 8, qui contient
a) 0,1 à 50 % en poids d'acide β-cétophosphonique de formule générale I,
b) 0,01 à 10 % en poids d'amorceur,
c) 0 à 80 % en poids d'un autre monomère,
d) 0 à 80 % en poids de charge,
e) 0 à 70 % en poids de solvant.

10. Matériau dentaire selon la revendication 9, pour utilisation en tant qu'adhésif, qui contient 0 à 20 % en poids de charge.

11. Matériau dentaire selon la revendication 10, qui contient
a) 0,1 à 50 % en poids, de préférence 1 à 40 % en poids et de façon particulièrement préférée 2 à 30 % en poids d'acides β-cétophosphoniques polymérisables de formule générale I,
b) 0,01 à 10 % en poids, de préférence 0,1 à 3,0 % en poids d'amorceur,
c) 0 à 60 %, de préférence 0 à 40 % en poids et de façon particulièrement préférée 5 à 40 % en poids de monomères mono- ou multifonctionnels non acides et/ou 0 à 60 % en poids et de préférence 0 à 30 % en poids de monomères acides,
d) 0 à 20 % en poids de charge,
e) 0 à 70 % en poids, de préférence 0 à 60 % en poids et de façon particulièrement préférée 0 à 50 % en poids de solvant.

12. Matériau dentaire selon la revendication 9, pour utilisation en tant que ciment ou matériau d'obturation, qui contient 20 à 80 % en poids de charge.

13. Matériau dentaire selon l'une quelconque des revendications 1 à 12, destiné à l'utilisation intra-orale en tant qu'adhésif, matériau d'obturation ou ciment.

14. Utilisation d'un matériau dentaire selon l'une quelconque des revendications 1 à 12, pour la réparation ou la réalisation extra-orale de restaurations dentaires.
